(19) Europäisches Patentamt
European Patent Office
Office européen des brevets

(11) Veröffentlichungsnummer: **0 046 193**
**B1**

# (12) EUROPÄISCHE PATENTSCHRIFT

(45) Veröffentlichungstag der Patentschrift: **28.09.83**

(51) Int. Cl.³: **C 07 D 231/12** // C07D261/08

(21) Anmeldenummer: **81105450.1**

(22) Anmeldetag: **13.07.81**

## (54) 1-Hydroxypyrazol und Verfahren zu seiner Herstellung.

(30) Priorität: **20.08.80 DE 3031385**

(43) Veröffentlichungstag der Anmeldung: **24.02.82 Patentblatt 82/8**

(45) Bekanntmachung des Hinweises auf die Patenterteilung: **28.09.83 Patentblatt 83/39**

(84) Benannte Vertragsstaaten: **BE CH DE FR GB LI**

(56) Entgegenhaltungen:
**AT-B-219 595**
**DE-A-2 031 489**
**DE-A-2 337 293**
**CHEMICAL ABSTRACTS, Band 61, Nr. 13, 21—12, 1964, Columbus, Ohio, USA, E. I. BUDOVSKII, J. A. HAINES, N. K. KOCHETKOW »Hydrazinolysis of pyrimidine nucleosides and DNA« Zusammenfassung Nr. 16 341 f**

(73) Patentinhaber: **BASF Aktiengesellschaft, Carl-Bosch-Strasse 38, D-6700 Ludwigshafen (DE)**

(72) Erfinder: **Rieber, Norbert, Dr., Liebfrauenstrasse 1C, D-6800 Mannheim (DE)**
Erfinder: **Boehm, Heinrich, Dr., Keplerweg 2, D-6701 Neuhofen (DE)**
Erfinder: **Platz, Rolf, Dr., Hansastrasse 5, D-6800 Mannheim (DE)**
Erfinder: **Fuchs, Werner, Dr., Muenchbuschweg 30B, D-6700 Ludwigshafen (DE)**

**EP 0 046 193 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

1-Hydroxypyrazol und Verfahren zu seiner Herstellung

Die Erfindung betrifft 1-Hydroxypyrazol und ein Verfahren zu seiner Herstellung durch Umsetzung von Isoxazolin-azoxi-verbindungen bei 140 bis 600° C.

1-Hydroxypyrazol ist bisher nur in Form von Derivaten mit mehreren Substituenten an den C-Atomen des Pyrazolringes bekannt. So wird in J. Org. Chem., Band 34, Seiten 194 bis 198 (1969) die Synthese von 3fach substituierten 1-Hydroxypyrazolen auf folgendem Wege beschrieben:

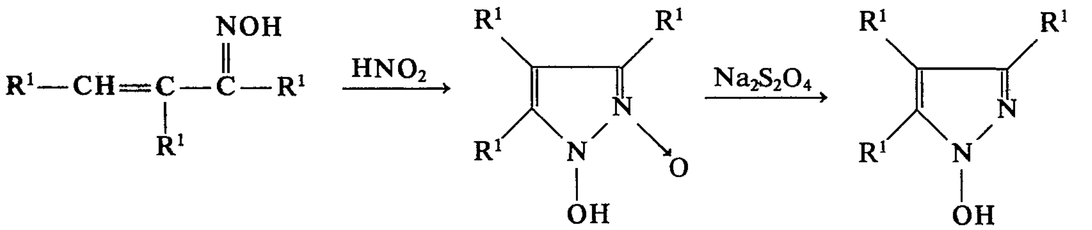


$R^1 = CH_3,\ C_6H_5$

Es wurde nun gefunden, daß man 1-Hydroxypyrazol der Formel

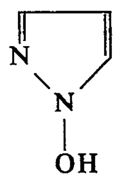

(Ia)

im Gemisch mit Isoxazolen der Formel

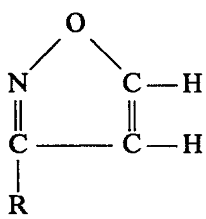

(Ib)

worin R ein Wasserstoffatom, einen aliphatischen, cycloaliphatischen, araliphatischen oder aromatischen Rest bedeutet, vorteilhaft erhält, wenn man Isoxazolin-azoxi-verbindungen der Formel

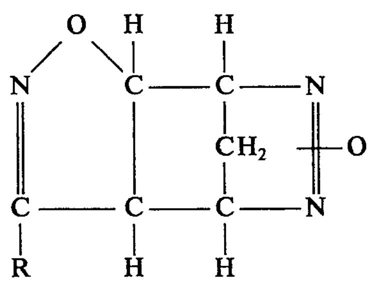

(II)

worin R die vorgenannte Bedeutung besitzt, bei einer Temperatur von 140 bis 600°C umsetzt.

Weiterhin wurde das neue 1-Hydroxypyrazol gefunden.

Die Umsetzung kann für den Fall der Verwendung von 2,3-Azoxi-6-oxa-7-aza-tricyclo-[5.2.1.0$^{5.9}$]-deca-2,7-dien durch die folgenden Formeln wiedergegeben werden:

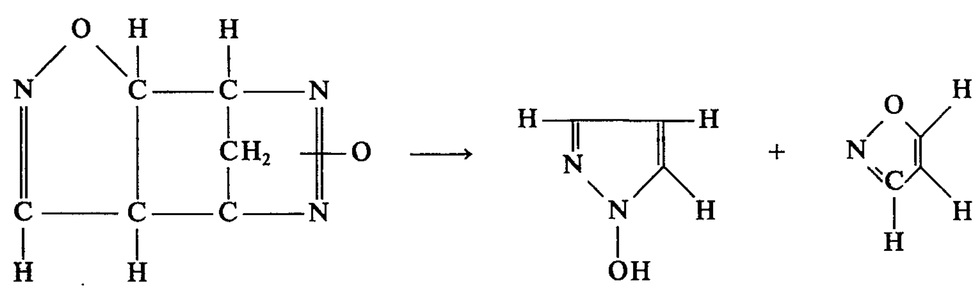

Das Verfahren nach der Erfindung liefert auf einfachem und wirtschaftlichem Wege das neue 1-Hydroxypyrazol sowie Isoxazole in guter Ausbeute und Reinheit. Alle diese vorteilhaften Ergebnisse sind mit Bezug auf den Stand der Technik überraschend.

Bevorzugte Ausgangsstoffe II und demzufolge bevorzugte Endstoffe Ia und Ib sind solche, in deren Formeln R ein Wasserstoffatom, einen Alkylrest mit 1 bis 18, insbesondere 1 bis 6 Kohlenstoffatomen, einen Cycloalkylrest mit 5 bis 8 Kohlenstoffatomen, einen Aralkylrest mit 7 bis 12 Kohlenstoffatomen, einen unsubstituierten oder durch Bromatome, Fluoratome, Chloratome, Alkylgruppen und/oder Alkoxygruppen mit 1 bis 4 Kohlenstoffatomen substituierten Phenylrest bedeutet. Die vorgenannten Reste können noch durch unter den Reaktionsbedingungen inerte Gruppen und/oder Atome, z. B. Alkylgruppen und Alkoxygruppen mit jeweils 1 bis 4 Kohlenstoffatomen, Phenylreste substituierende Bromatome, Fluoratome, Chloratome, substituiert sein. Die Ausgangsstoffe II können leicht durch Umsetzung von 2,3,7-Triaza-6-oxa-tricyclo-[5.2.1.0$^{5.9}$]-deca-2,7-dienen der Formel

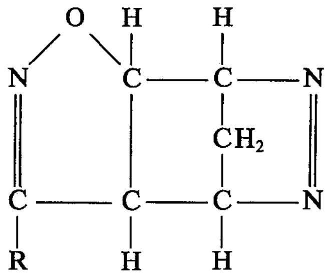

(III)

worin R die vorgenannte allgemeine und bevorzugte Bedeutung besitzt, mit einem organischen Peroxid bei einer Temperatur von $-10$ bis $+130°$C, vorzugsweise 20 bis 100°C, insbesondere 50 bis 90°C, drucklos oder unter Druck, kontinuierlich oder diskontinuierlich, in Abwesenheit oder Anwesenheit von organischen Lösungsmitteln wie Halogenkohlenwasserstoffe und Äther, erhalten werden. Das Peroxid kann in stöchiometrischer Menge oder im Überschuß, bevorzugt in einem Verhältnis von 1 bis 10, insbesondere 1 bis 1,5 Mol Peroxid je Mol Ausgangsstoff umgesetzt werden. Die Ausgangsstoffe III ihrerseits können nach folgender Arbeitsweise hergestellt werden:

Die Ausgangsstoffe III können leicht durch Umsetzung in einem ersten Schritt von Nitriloxiden der Formel

$R^1CNO$ (IV)

worin $R^1$ die vorgenannte allgemeine und bevorzugte Bedeutung besitzt, mit N,N,'-Dicarbalkoxy-2,3-diaza-bicyclo-[2,2,1]-hept-2-enen der Formel

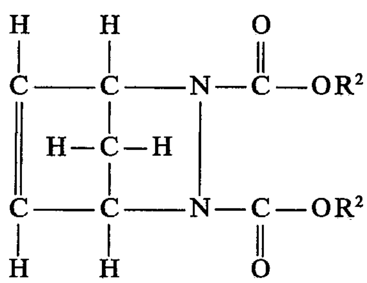

(V)

worin die einzelnen Reste $R^2$ gleich oder verschieden sein können und jeweils ein Wasserstoffatom oder einen aliphatischen Rest bedeuten, und dann in einem 2. Schritt die so erhaltenen N,N'-Dicarbalkoxy-isoxazolino-verbindungen der Formel

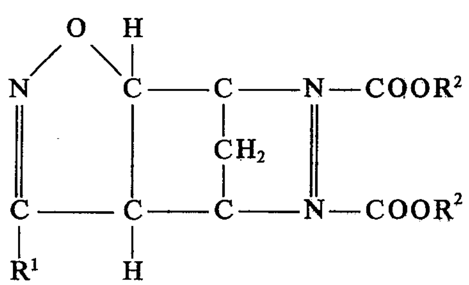

(VI)

worin $R^1$ die vorgenannte Bedeutung besitzt, nach üblicher Methode verseift, decarboxyliert und zu den Ausgangsstoffen III oxidiert.

Die Ausgangsstoffe IV sind leicht durch die in Houben—Weyl, Methoden der Organischen Chemie,

Band 10/3, Seiten 841 bis 853, beschriebenen Arbeitsweise, z. B. durch Dehydrierung von Aldoximen oder aus Hydroxamsäurederivaten oder Nitrolsäuren zugänglich. Die Ausgangsstoffe V erhält man z. B. durch Umsetzung von Cyclopentadien mit Azodicarbonsäuredimethylestern nach den in Ann. 443, 242 bis 262 (1925) beschriebenen Verfahren. Der Sauerstoff der Azoxygruppe kann sowohl am einen wie am anderen Stickstoffatom der Azogruppierung gebunden sein. Daher können als Ausgangsstoffe II die reinen Isomere

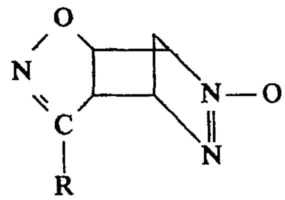

und

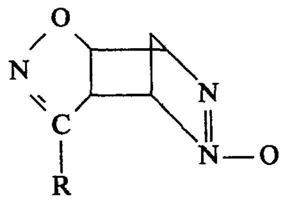

oder zweckmäßig das Isomerengemisch, wie es bei der Herstellung anfällt, verwendet werden.

So kommen beispielsweise die folgenden Ausgangsstoffe II infrage: 2,3-Azoxi-6-oxa-7-aza-tricyclo-5.2.1.$0^{5.9}$-deca-2,7-dien und seine in 8-Stellung durch die Methyl-, Äthyl-, Propyl-, Isopropyl-, Butyl-, Isobutyl-, sek.-Butyl-, tert.-Butyl-, Cyclohexyl-, Cyclopentyl-, Benzyl-, Phenyl-, 2'-Chlorphenyl-, 3'-Chlorphenyl-, 4'-Chlorphenyl-, 2'-Methylphenyl-, 3'-Methylphenyl-, 4'-Methylphenyl-, 2'-Methoxyphenyl-, 3'-Methoxyphenyl-, 4'-Methoxyphenyl-, 2'-Äthylphenyl-, 3'-Äthylphenyl-, 4'-Äthylphenyl-, 2'-Äthoxyphenyl-, 3'-Äthoxyphenyl-, 4'-Äthoxyphenyl-gruppe substituierten Homologen.

Die Umsetzung wird bei einer Temperatur von 140 bis 600°C, vorzugsweise 150 bis 500°C, im Falle von Ausgangsstoffen II mit R in der Bedeutung Wasserstoff, aliphatischem, cycloaliphatischem, araliphatischem Rest zweckmäßig bei einer Temperatur von 200 bis 600, vorzugsweise 250 bis 500, insbesondere 300 bis 450°C, im Falle von Ausgangsstoffen II mit R in der Bedeutung aromatischem Rest von 140 bis 200°C, vorzugsweise 150 bis 190°C, insbesondere 160 bis 180°C, drucklos, mit Unterdruck oder unter Druck, diskontinuierlich oder kontinuierlich durchgeführt. Man kann unter den Reaktionsbedingungen inerte, organische Lösungsmittel verwenden oder schon aus wirtschaftlichen Gründen zweckmäßig in Abwesenheit organischer Lösungsmittel umsetzen.

Die Reaktion kann wie folgt durchgeführt werden: Der Ausgangsstoff II wird während einer Sekunde bis 21 Stunden bei der Reaktionstemperatur gehalten. Dann werden die Endstoffe Ia und Ib aus dem Reaktionsgemisch in üblicher Weise, z. B. durch fraktionierte Destillation bzw. Kondensation, Extraktion oder Kristallisation, abgetrennt.

Die nach dem Verfahren der Erfindung herstellbaren Endstoffe Ia und Ib sind wertvolle Ausgangsstoffe für die Herstellung von Farbstoffen, Pflanzenschutzmitteln und Pharmazeutika. 1-Hydroxypyrazol kann leicht analog der in J. Org. Chem., loc. cit., beschriebenen Arbeitsweise durch Reduktion mit Zink oder durch Hydrierung mit Raney-Nickel in Pyrazol überführt werden. Bezüglich der Verwendung wird auf die vorgenannte Veröffentlichung und Ullmanns Encyklopädie der technischen Chemie, Band 8, Seiten 498 bis 500, verwiesen. Die Verwendung von Pyrazol als Ausgangsstoff zur Herstellung von Herbiziden ist in der DE-AS 2 648 008 beschrieben.

Die in den folgenden Beispielen angeführten Teile bedeuten Gewichtsteile.

## Beispiel 1

97 Teile 8-Methyl-2,3-azoxi-6-oxa-7-aza-tricyclo-[5,2,1,$0^{5.9}$]-decy-2,7-dien werden innerhalb von 2 Stunden bei 455°C und 5 mbar durch einen mit 300 Teilen Quarzringe gefüllten Rohrreaktor geführt. Das Reaktionsgemisch wird in 2 Vorlagen fraktioniert kondensiert. In der ersten auf 5°C gekühlten Vorlage erhält man 47 Teile (97% der Theorie) 1-Hydroxypyrazol vom Fp 75°C (aus Ligroin). Die zweite auf −70°C gekühlte Vorlage enthält 46 Teile (95% der Theorie) 3-Methylisoxazol vom Kp 117°C.

## Beispiel 2

100 Teile 8-Phenyl-2,3-azoxi-6-oxa-7-aza-tricyclo-[5.2.1.$0^{5.9}$]-deca-2,7-dien werden 20 Stunden auf 170°C erhitzt. Anschließend wird das Reaktionsgemisch bei 5 mbar destilliert ($Kp_{5\,mbar}$ 50 bis 120°C), das Destillat mit 200 Teilen Wasser versetzt und das Gemisch zweimal mit 100 Teilen Petroläther extrahiert. Durch Einengen der beiden Phasen im Rotationsverdampfer bei 10 bis 20 mbar und 20 bis 40°C Badtemperatur erhält man aus der Wasserphase 25 Teile (68% der Theorie) 1-Hydroxypyrazol vom Fp 75°C und aus der organischen Phase 55 Teile (87% der Theorie) 3-Phenyl-isoxazol vom $Kp_{(20\,mbar)}$ 130°C.

Beispiel 3

10 Teile 8-(p-Chlorphenyl)-2,3-azoxi-6-oxa-7-aza-tricyclo-[5.2.1.$0^{5.9}$]-deca-2,7-dien werden 10 Stunden auf 170°C erhitzt und anschließend bei 10 bis 20 mbar und einer Badtemperatur bis zu 160°C sublimiert. Man erhält (bestimmt durch NMR-Analyse) 2,85 Teile (89% der Theorie) 1-Hydroxypyrazol vom Fp 75°C und 6,33 Teile (93% der Theorie) 3-(p-Chlorphenyl-)-isoxazol vom Fp 78°C. Die Schmelzpunkte wurden nach säulenchromatographischer Trennung des Gemischs an Kieselgel (Laufmittel: Petroläther/Äther) bestimmt.

Beispiel 4

35 Teile 8-Äthyl-2,3-azoxi-6-oxa-7-aza-tricyclo-[5.2.1.$0^{5.9}$]-deca-2,7-dien werden innerhalb von 1,5 Stunden bei 410°C und 65 mbar durch einen mit 300 Teilen Quarzringe gefüllten Rohrreaktor geführt. Das Reaktionsgemisch wird in einer Vorlage bei −60°C kondensiert. Aus dem Kondensat erhält man durch Destillation 14,6 Teile (78% der Theorie) 3-Äthylisoxazol vom Kp 133°C und 14,8 Teile (91% der Theorie) 1-Hydroxypyrazol vom Fp 75°C (aus Ligroin).

Beispiel 5

25 Teile 8-Isopropyl-2,3-azoxi-6-oxa-7-aza-tricyclo-[5.2.1.$0^{5.9}$]-deca-2,7-dien werden, wie in Beispiel 2 beschrieben, innerhalb von einer Stunde umgesetzt. Durch Destillation des Kondensats erhält man 10,1 Teile (94% der Theorie) 1-Hydroxypyrazol vom Fp 75°C (aus Ligroin) und 13,1 Teile (92% der Theorie) 3-Isopropylisoxazol vom Kp 144°C.

Beispiel 6

43 Teile 8-tert.-Butyl-2,3-azoxi-6-oxa-7-aza-tricyclo-[5.2.1.$0^{5.9}$]-deca-2,7-dien werden, wie in Beispiel 2 beschrieben, innerhalb von 1,5 Stunden umgesetzt. Man erhält durch Destillation des Kondensats 17 Teile (93% der Theorie) 1-Hydroxypyrazol vom Fp 75°C (aus Ligroin) und 23 Teile (93,5% der Theorie) 3-t-Butylisoxazol vom $Kp_{40}$mbar 75°C.

Beispiel 7 (Verwendung)

Ein Teil 1-Hydroxypyrazol wird in 10 Teilen 32gewichtsprozentiger Salzsäure gelöst und bei 60°C portionsweise mit 5 Teilen Zink versetzt. Nach 16 Stunden wird die Reaktionslösung mit Natronlauge neutralisiert und mit Äther extrahiert. Man erhält durch Einengen der Ätherphase 0,6 Teile Pyrazol (75% der Theorie) vom Fp 68°C (aus Ligroin).

Beispiel 8 (Verwendung)

Ein Teil 1-Hydroxypyrazol und 0,3 Teile Raney-Nickel in 10 Teilen Methanol werden bei 100°C 5 Stunden mit 50 bar Wasserstoff umgesetzt. Es wird vom Katalysator abfiltriert, mit Methanol nachgewaschen und die Lösung eingedampft. Man erhält 0,75 Teile Pyrazol (93% der Theorie) vom Fp 68°C (aus Ligroin).

**Patentansprüche**

1. 1-Hydroxypyrazol der Formel

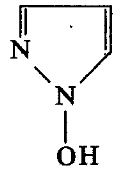

(Ia)

2. Verfahren zur Herstellung von 1-Hydroxypyrazol der Formel

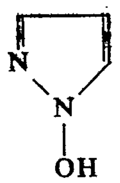

(Ia)

im Gemisch mit Isoxazolen der Formel

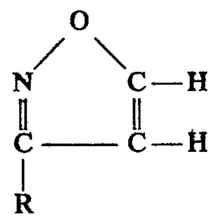

(Ib)

worin R ein Wasserstoffatom, einen aliphatischen, cycloaliphatischen, araliphatischen, aromatischen Rest bedeutet, wobei man Isoxazolin-azoxi-verbindungen der Formel

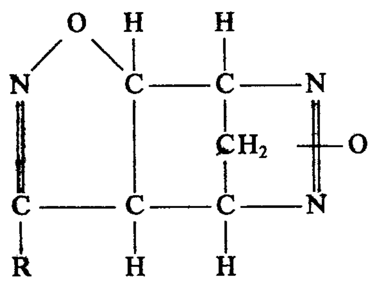

(II)

worin R die vorgenannte Bedeutung besitzt, bei einer Temperatur von 140 bis 600°C umsetzt.

**Claims**

1. 1-Hydroxypyrazole of the formula

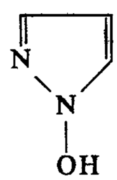

(Ia)

2. A process for the preparation of 1-hydroxypyrazole of the formula

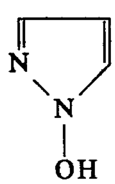

(Ia)

mixed with isoxazoles of the formula

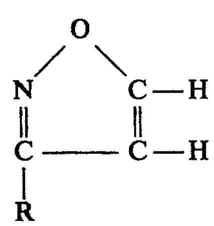

(Ib)

where R is hydrogen or an aliphatic, cycloaliphatic, araliphatic or aromatic radical, wherein an isoxazoline-azoxy compound of the formula

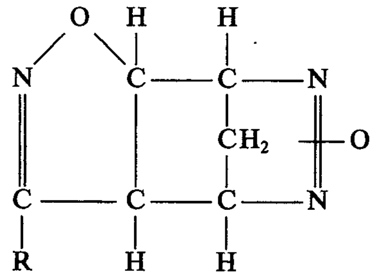

(II)

where R has the above meaning, is converted at from 140 to 600°C.

**Revendications**

1. 1-Hydroxy-pyrazole de la formule

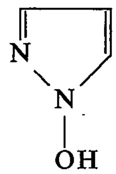

(Ia)

2. Procédé de préparation du 1-hydroxy-pyrazole de la formule

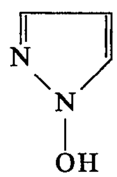

(Ia)

en mélange avec des isoxazoles de la formule

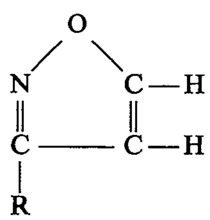

(Ib)

dans laquelle R désigne un atome d'hydrogène ou un groupe aliphatique, cycloaliphatique, araliphatique ou aromatique, dans lequel on fait réagir à une température comprise entre 140 et 600°C des dérivés azoxy d'isoxazolines de la formule

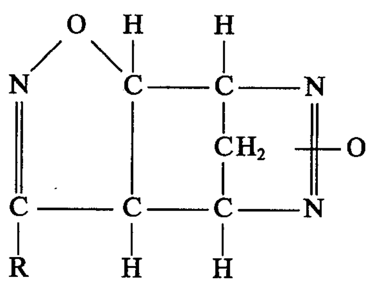

(II)

dans laquelle R possède la signification définie.